# EUROPÄISCHE PATENTSCHRIFT

(11) **EP 2 379 696 B1**
(45) Veröffentlichungstag und Bekanntmachung des Hinweises auf die Patenterteilung: **02.01.2019**
(21) Anmeldenummer: 10703028.0
(22) Anmeldetag: 19.01.2010
(51) Int. Cl.: C12M 1/107, C12P 5/02

(54) **BIOGAS-ANLAGE MIT ZUSTANDSKONTROLLE FÜR DIE FERMENTIERUNG**
BIOGAS PLANT HAVING STATE MONITORING FOR FERMENTATION
INSTALLATION DE PRODUCTION DE BIOGAZ À CONTRÔLE DE DÉROULEMENT DE LA FERMENTATION

(30) Priorität: 20.01.2009 DE 102009005560; 21.09.2009 DE 102009042418
(43) Veröffentlichungstag der Anmeldung: 26.10.2011
(73) Patentinhaber: Siemens Aktiengesellschaft, 80333 München (DE)
(72) Erfinder: FLEISCHER, Maximilian, 85635 Höhenkirchen (DE); GUIJARRO-REZNICKOVA, Jarmila, 81543 München (DE); KUBISCH, Rebekka, 85368 Moosburg an der Isar (DE); PASTUSIAK, Remigiusz, 81549 München (DE); WIESNER, Kerstin, 85640 Putzbrunn (DE)
(86) Internationale Anmeldenummer: PCT/EP2010/050548
(87) Internationale Veröffentlichungsnummer: WO 2010/084108

(56) Entgegenhaltungen:
- DE-A1- 3 102 739
- Phil Hobbs et al.: "Optimisation of biogas production from a microbial perspective" In: "13th RAMIRAN International Conference" Juni 2008 (2008-06), Ministry of Agriculture and Food. Agricultural Academy , Albena (Bulgaria) , XP002587958 , Seiten 327-330 das ganze Dokument
- INGVAR SUNDH ET AL.: "Effects of glucose overloading on microbial community structure and biogas production in a laboratory-scale anaerobic digester" BIORESOURCE TECHNOLOGY, Bd. 89, 2003, Seiten 237-243, XP002587959
- IMAN W. KOSTER ET AL.: "Inhibition of Methanogenesis from Acetate in Granular Sludge by Long Chain Fatty Acids" APPLIED AND ENVIRONMENTAL MICROBIOLOGY, Bd. 53, Nr. 2, Februar 1987 (1987-02), Seiten 403-409, XP002587960

## Beschreibung

Für Biogas-Anlagen ist es wichtig, dass die Fermentation in der richtigen Weise abläuft. Daher werden auch moderne Biogasanlagen heutzutage aufgrund fehlender Messtechnik überwiegend im Unterlastbetrieb gefahren, um Instabilitäten und im Extremfall ein Kippen des Fermentationsprozesses zu verhindern. Daraus resultiert eine reduzierte Energieausbeute. Ein großes Problem dabei ist die Heterogenität der Rohsubstrate - derzeit überwiegend Silage und Gülle - denn die Dosierung der Substrate auf Basis der Frischmasse führt zu erheblichen Variationen der zugeführten Nährstofffracht. Diese Variationen führen zu einem Ungleichgewicht im Gesamtprozess, da der enzymatische und bakterielle Abbau der komplexen Polymere zu einer Anreicherung von kurzkettigen organischen Säuren führen kann, wenn diese nicht hinreichend schnell durch die methanbildenden Mikroorganismen verbraucht werden. Hohe Säurekonzentration, vor allem der Essig- und Propionsäure, hemmen die Methanbildung und können im Extremfall zu einem Absterben der entsprechenden Bakterien-Populationen führen.

Derzeit erfolgt die Überwachung des Fermenterprozesses von Biogas-Anlagen in den meisten Fällen stichprobenartig. Dabei werden Proben des Fermenterinhalts in bestimmten Abständen durch ein externes Analytiklabor untersucht. Die Untersuchung konzentriert sich dabei auf den Gehalt der Probe an niedermolekularen organischen Säuren. Diese Untersuchungsmethodik basiert darauf, dass sich die säurebildenden Bakterien zum Teil sehr schnell vermehren können, während sich die methanbildenden Bakterien nur langsam vermehren. Eine Zugabe von chemisch leicht aufschließbarem Material kann daher zu einer raschen Absenkung des pH-Werts führen. Die methanbildenden Bakterien reagieren darauf empfindlich. Zwar stellen die Säuren für sie das Substrat dar, doch führt ein Überangebot davon tatsächlich zu einer Leistungshemmung. Im Extremfall wird sogar eine Einstellung der Aktivität, d.h. der Methanbildung bewirkt, was als "Umkippen" der Fermentation bezeichnet wird.

Eine Überwachung basierend auf der chromatografischen Analyse von PLFA (phospholipid fatty acids) ist in Phil Hobbs et al., "Optimisation of biogas production from a microbial perspective", 13th RAMIRAN Int. Conf., 2008, S. 327-330 beschrieben.

Es ist Aufgabe der vorliegenden Erfindung, ein verbessertes Verfahren anzugeben, eine Biogas-Anlage auf ein Ungleichgewicht, speziell die Übersäuerung, in der Fermentation zu überwachen. Eine weitere Aufgabe besteht darin, eine entsprechend ausgestaltete Biogas-Anlage anzugeben.

Hinsichtlich des Verfahrens wird die Aufgabe durch ein Verfahren mit den Merkmalen von Anspruch 1 gelöst. Hinsichtlich der Biogas-Anlage wird die Aufgabe durch eine Biogas-Anlage mit den Merkmalen von Anspruch 8 gelöst. Die abhängigen Ansprüche beziehen sich auf vorteilhafte Ausgestaltungen und Weiterbildung der Erfindung.

Bei dem erfindungsgemäßen Verfahren zur Detektion eines Ungleichgewichts der Fermentation im Fermenter in einer Biogas-Anlage wird eine erste Menge von grampositiven Bakterien in wenigstens einen Teils des Fermenterinhalts ermittelt und aus der ersten Menge von grampositiven Bakterien bestimmt, ob ein Ungleichgewicht der Fermentation vorliegt.

Die erfindungsgemäße Lösung der Aufgabe basiert auf folgender Annahme, die mittels Untersuchungen verifiziert werden konnte: es besteht eine Analogie zwischen der Mikroorganismenflora des Pansens eines Wiederkäuers und dem Fermenterreaktor einer Biogas-Anlage. Im weitaus besser untersuchten und studierten Pansen wurde aus tiermedizinischen Studien folgende bekannte bakterielle Zusammensetzung belegt: bei einem gesunden Tier mit einer gemischten Rationsfütterung ist die Pansenflora überwiegend gramnegativ und durch eine Vielfalt der Formen gekennzeichnet, während bei einer fehlerhaften Fütterung, beispielsweise einem zu hohen Anteil an leicht verdaulichen Kohlenhydraten, diese Vielfalt abnimmt und bald grampositive, milchsäureproduzierende Streptokokken und Laktobazillen dominieren. Letzteres ist als Pansenazidose-Krankheit bekannt. Eine analoge Veränderung der Bakterienpopulation im Fermenter wird erfindungsgemäß als Indikator für eine Prozessstörung in der Biogas-Anlage verwendet.

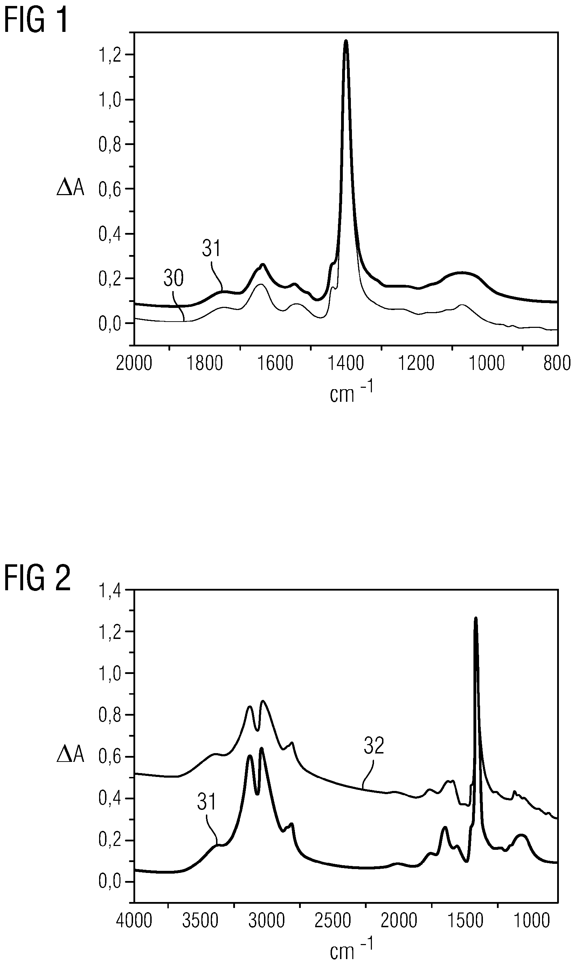

Im Sinne eines Beispiels zeigt Figur 1 ein MIR-Spektrum 30, MIR = mittleres Infrarot, von E.coli, einer repräsentativen Kultur für gramnegative Bakterien in einem Puffer (Ringerlösung). Die Probe wurde auf einem Zinkselenidkristall als Film mit der Attenuated Total Reflection-Technik im Wellenlängenbereich von 800-2000 cm⁻¹ gemessen. Das Lösungsmittel, in diesem Fall Ringerpuffer, wurde als Hintergrund vom Absolutspektrum abgezogen. Zusätzlich zeigt Figur 1 ein analoges Spektrum 31, das mit der gleichen Methode und Auswertung erstellt wurde. In diesem Fall wurde jedoch eine Fermenter-Probe, ebenfalls in Ringer-Lösung verdünnt, gemessen. Diese Probe wurde aus einem einwandfreien Fermentationsreaktor entnommen. Die Ähnlichkeit zwischen den beiden Spektren 30, 31 der Figur 1 ist unverkennbar.

Figur 2 zeigt wiederum das Spektrum 31 der Fermenter-Probe. Zusätzlich zeigt Figur 2 ein Spektrum 32 einer "umgekippten" Fermenter-Probe. Diese wurde simuliert, indem einer frischen Fermenterprobe ein Überschuss an Stärke als leicht verdaulichem Substrat hinzugefügt wurde. In einem parallelen Versuch wurde einer anderen frischen Fermenterprobe direkt ein Überschuss an Glukose hinzugefügt. Glukose stellt ein wichtiges Endprodukt der ersten Abbauprozesse, der Hydrolyse, dar. Nach einer mehrtägigen Inkubation dieser behandelten Fermenterproben bei 39 °C wurden diese wie oben beschrieben spektroskopisch vermessen. Figur 2 zeigt den Unterschied zwischen den umgekippten Fermenterproben und den neutralen Fermenterproben deutlich.

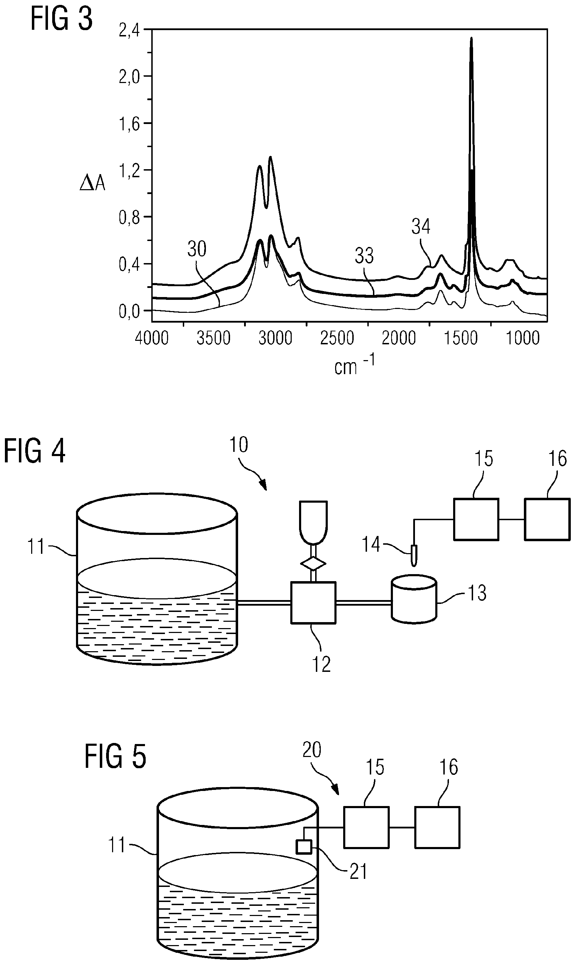

Um sicherzustellen, dass die spektralen Unterschiede auf einer tatsächlichen unterschiedlichen Präsenz der beiden Bakterienarten beruhen und nicht ein Artefakt die jeweiligen Probe darstellen, wurden in der Petrischale auf speziellen Nährböden die beiden aktiven Sorten kultiviert. Mit zusätzlichen biochemischen Verfahren, beispielsweise Kolonieauszählung und Farbindikatoren wurde sichergestellt, dass die vorgesehene Kultivierung auch erreicht wurde. Diese definierten Proben wurden ebenfalls spektroskopiert und es zeigen sich die gleichen Veränderungen im Spektrum wie bei den Fermenterproben, siehe Figur 3. Figur 3 zeigt wiederum das Spektrum 30 der E.coli-Bakterien und zusätzlich ein Spektrum 33 der gramnegativen und ein Spektrum 34 der grampositiven Bakterien.

Erfindungsgemäß werden also vorteilhaft nicht die kumulierten Abbauprodukte, d.h. die organischen Säuren, sondern direkt deren Verursacher, also die Menge der Bakterien selbst, detektiert. Das ermöglicht ein frühzeitiges Entgegenwirken bei einer Prozessstörung. Dadurch wird nicht nur das Umkippen der Fermentation verhindert. Es wird auch möglich, den Fermentationsprozess allgemein optimaler zu gestalten, da Sicherheitsmargen bei der Gestaltung des Prozesses verringert werden können. Dabei kann vorteilhaft kontinuierlich auf die Menge an grampositiven Bakterien oder das Verhältnis zu den gramnegativen Bakterien geachtet und auf die Fermentation eingewirkt werden.

Bevorzugt wird zusätzlich eine zweite Menge von gramnegativen Bakterien in wenigstens einem Teil des Fermenterinhalts ermittelt. Diese zweite Menge kann vorteilhaft zusammen mit der ersten Menge dazu verwendet werden, zu bestimmen, ob ein Ungleichgewicht der Fermentation vorliegt. Dazu kann beispielsweise das Verhältnis der ersten Menge und der zweiten Menge zueinander verwendet werden.

Eine Möglichkeit zur Bestimmung der ersten Menge und gegebenenfalls der zweiten Menge besteht in einer labortechnischen Untersuchung einer Probe aus dem Fermenterinhalt. Dabei kommen bevorzugt mikrobiologische Verfahren zur Verwendung, beispielsweise ein Gram-Test, eine Kultivierung auf selektiven Nährböden oder eine Auswertung der Zellmorphologie. Dabei werden die Proben zur Untersuchung zweckmäßig regelmäßig entnommen und, ebenso wie bei der Untersuchung mittels Infrarotspektroskopie die Menge oder Mengen der grampositiven und gramnegativen Bakterien oder das Verhältnis der Mengen oder ein aus den Einzelwerten abgeleiteter Wert betrachtet und ausgewertet.

Erfindungsgemäß passiert die Ermittlung der ersten Menge und gegebenenfalls der zweiten Menge direkt im Fermenter. Dabei kann insbesondere vorteilhaft eine Online-Untersuchung stattfinden, das heißt die ermittelten ersten und zweiten Mengen stehen zeitnah zur Verfügung. Dadurch kann vorteilhaft ausreichend schnell in den Arbeitsablauf des Fermenters eingegriffen werden, um ein Umkippen des Fermentationsprozess zu verhindern. Erfindungsgemäß wird zur Bestimmung der ersten Menge und gegebenenfalls der zweiten Menge Infrarotspektroskopie verwendet. Dabei können sowohl eine Infrarotspektroskopie im nahen Infrarotbereich (NIR) als auch im mittleren Infrarotbereich (MIR) verwendet werden.

Bei der Verwendung des mittleren Infrarotbereichs können zur Signalextraktion regelbasierte analytische Verfahren angewendet werden. Diese sind im Fall des nahen Infrarotbereichs schlecht anwendbar, da eindeutige chemischen Spezies zuzuordnende spektrale Strukturen hier nicht vorliegen. In diesem Fall sind die Methoden der statistischen Mathematik im Sinne einer sogenannten multivariaten Signalverarbeitung anzuwenden. Diese werten die komplette Form des eingegebenen Spektrums aus, ohne das bekannt sein muss, in welchen Bereichen die relevante Information enthalten ist. Zu diesen gehören beispielsweise die Hauptkomponentenanalyse (PCA), lineare Regression (LDA), partial least square Verfahren (PLS oder PLSDA), wie auch die Methode der neuronalen Netzwerke (ANN). Zur Reduktion der benötigten Rechenzeit wie auch zur Erhöhung der Empfindlichkeit gegenüber Störungen können hierbei nur vorselektive Bereiche des Spektrums der Auswertung zugeführt werden. Vor diesen statistischen Verfahren wird günstigerweise eine Signalvorverarbeitung (Glättung, Normierung bzw., die Elimination von Messausreißern) durchgeführt.

Die Auswertung der Messergebnisse einer Infrarotsensorik findet zweckmäßig außerhalb des Fermenters statt. Die Sensorik selbst, also die Anordnung des eigentlichen Infrarot-Sensors, kann jedoch innerhalb des Fermenters oder außerhalb des Fermenters stattfinden. Für eine Verwendung innerhalb des Fermenters kommt dabei bevorzugt eine Reflexions-Sonde zum Einsatz. Bei dieser Art von Messung ist es zweckmäßig, die Reflexions-Sonde zu eichen und Referenzspektren aufzunehmen. Dafür besteht der Vorteil einer Messung direkt im Fermenter darin, eine automatisierte Online-Überwachung des Fermentationsprozesses zu ermöglichen.

Bei einer Sensorik außerhalb des Fermenters kann beispielsweise eine Probenentnahme erfolgen, wobei die entnommene Probe verdünnt wird und bevorzugt mittels einer Durchfluss-Sonde vermessen wird. Die Signale der Durchfluss-Sonde wobei eine Durchstrahlungs- oder aber auch Reflexions-Geometrie verwendet wird, werden dabei zweckmäßig an ein Spektrometer übermittelt und mittels eines Mikroprozessors oder eines Computers ausgewertet. Der Vorteil einer Messung außerhalb des Fermenters besteht darin, dass die meisten Teile der Sensoranordnung sich außerhalb des Fermenters und somit im leichten Zugriff von außen befinden.

Bevorzugte, jedoch keinesfalls einschränkende Ausführungsbeispiele für die Erfindung werden nunmehr anhand der Zeichnung näher erläutert. Dabei sind die Merkmale schematisiert dargestellt und sich entsprechende Merkmale sind mit gleichen Bezugszeichen markiert. Die Figuren zeigen dabei im Einzelnen
Figur 4 eine Biogas-Anlage mit einer externen Sensoranordnung und
Figur 5 eine Biogas-Anlage mit einer internen Sensoranordnung.

Eine erste Biogasanlage 10 gemäß einem ersten Ausführungsbeispiel, dargestellt in Figur 4, umfasst einen Fermenter 11, einen Mixer 12, eine Tauchsonde 14 und ein Tauchbehältnis 13, ein Spektrometer 15 und einen Auswerterechner 16. Bei der ersten Biogasanlage 10 findet die eigentliche Infrarotmessung außerhalb des Fermenters 11 statt. Der eigentlichen Messung sind dabei zwei Schritte vorgelagert, die der Probenvorbereitung dienen. Dabei wird im Mixer 12 eine im ersten Schritt aus dem Fermenter 11 entnommene Probe des Fermenterinhalts zerkleinert und homogenisiert und im zweiten Schritt verdünnt. Die verdünnte Probe des Fermenterinhalts wird im Tauchbehältnis 13 für die Tauchsonde 14 bereitgehalten. Das Messergebnis der Tauchsonde 14 wird an das Spektrometer 15 weitergegeben. Bei dem Spektrometer 15 handelt es sich in diesem Ausführungsbeispiel um ein prozessfähiges FT-MIR-Spektrometer in Verbindung mit einer fasergekoppelten Tauchsonde 14.

Das Spektrometer 15 liefert die sich ergebenden Infrarotspektren an den Auswerterechner 16. Der Auswerterechner 16 führt einen Vergleich der Infrarotspektren mit gespeicherten Referenzspektren von grampositiven und gramnegativen Proben durch und ermittelt daraus, ob ein Ungleichgewicht im Fermenter 11 vorliegt.

In einer zweiten Biogasanlage 20 gemäß einem zweiten Ausführungsbeispiel, dargestellt in Figur 5, wird die externe Probenprozessierung vermieden. Dazu ist in diesem Fall der Sensor, im zweiten Ausführungsbeispiel eine Reflexions-Sonde 21, in den Fermenter 11 direkt integriert. Die Reflexions-Sonde 21 ist mit dem Spektrometer 15 verbunden. Analog zum ersten Ausführungsbeispiel liefert auch hier das Spektrometer 15 Spektrallinieninformationen an den Auswerterechner 16, der daraus durch einen Vergleich auf ein Ungleichgewicht im Fermenter 11 schließt.

## Patentansprüche

1. Verfahren zur Detektion eines Ungleichgewichts der Fermentation in einer Biogas-Anlage (10, 20) mit einem Fermenter (11), bei dem mittels Infrarotspektroskopie eine erste Menge von grampositiven Bakterien in wenigstens einem Teil des Fermenterinhalts ermittelt wird und aus der ersten Menge von grampositiven Bakterien bestimmt wird, ob ein Ungleichgewicht der Fermentation vorliegt, wobei die Bestimmung der Menge direkt im Fermenterinhalt stattfindet.

2. Verfahren gemäß Anspruch 1, bei dem zusätzlich eine zweite Menge von gramnegativen Bakterien in wenigstens einem Teil des Fermenterinhalts ermittelt wird.

3. Verfahren gemäß Anspruch 2, bei dem aus dem Verhältnis der ersten Menge und der zweiten Menge bestimmt wird, ob ein Ungleichgewicht der Fermentation vorliegt.

4. Biogas-Anlage (10, 20) mit einem Fermenter (11), ausgestaltet zur Detektion eines Ungleichgewichts der Fermentation im Fermenter (11), umfassend eine im Fermenter (11) angeordnete Sensoranordnung (12...16, 21) zur Bestimmung einer ersten Menge von grampositiven Bakterien in wenigstens einem Teil des Fermenterinhalts mittels Infrarot-Spektroskopie.

5. Biogas-Anlage (10, 20) gemäß Anspruch 4, bei der die Sensoranordnung (12...16, 21) ausgestaltet ist, zusätzlich eine zweite Menge von gramnegativen Bakterien in wenigstens einem Teil des Fermenterinhalts zu bestimmen.

6. Biogas-Anlage (10, 20) gemäß Anspruch 4 oder 5, bei der die Sensoranordnung (12...16, 21) eine Reflexionssonde (21) im Inneren des Fermenters (11) umfasst.

7. Biogas-Anlage (10, 20) gemäß einem der Ansprüche 4 bis 6, bei der die Sensoranordnung (12...16, 21) ausgestaltet ist, die Infrarot-Spektroskopie im Bereich des Nahinfrarotspektrums durchzuführen.

8. Biogas-Anlage (10, 20) gemäß einem der Ansprüche 4 bis 7, bei der Mittel für eine statistische Datenauswertung für die spektroskopischen Daten vorgesehen sind.

## Claims

1. Method for detecting an imbalance in the fermentation in a biogas plant (10, 20) with a fermenter (11), in which a first quantity of gram-positive bacteria is ascertained in at least one part of the fermenter content using infrared spectroscopy and the first quantity of gram-positive bacteria is used to determine whether an imbalance exists in the fermentation, wherein the determination of the quantity takes place directly in the fermenter content.

2. Method according to claim 1, in which a second quantity of gram-negative bacteria is additionally ascertained in at least one part of the fermenter content.

3. Method according to claim 2, in which the ratio of the first quantity to the second quantity determines whether an imbalance exists in the fermentation.

4. Biogas plant (10, 20) with a fermenter (11), configured to detect an imbalance in the fermentation in the fermenter (11), comprising a sensor arrangement (12...16, 21) arranged in the fermenter (11) for determining a first quantity of gram-positive bacteria in at least one part of the fermenter content using infrared spectroscopy.

5. Biogas plant (10, 20) according to claim 4, in which the sensor arrangement (12...16, 21) is configured additionally to determine a second quantity of gram-negative bacteria in at least one part of the fermenter content.

6. Biogas plant (10, 20) according to claim 4 or 5, in which the sensor arrangement (12...16, 21) comprises a reflection probe (21) inside the fermenter (11).

7. Biogas plant (10, 20) according to one of claims 4 to 6, in which the sensor arrangement (12...16, 21) is configured to carry out the infrared spectroscopy in the region of the near-infrared spectrum.

8. Biogas plant (10, 20) according to one of claims 4 to 7, in which means are provided for a statistical data evaluation of the spectroscopic data.

## Revendications

1. Procédé de détection d'un déséquilibre de la fermentation dans une installation (10, 20) de production de biogaz, comprenant un fermenteur (11), dans lequel, au moyen de la spectroscopie infrarouge, on détermine un premier nombre de bactéries prenant le gram, dans au moins une partie du contenu du fermenteur et, à partir du premier nombre de bactéries prenant le gram, on détermine s'il y a un déséquilibre de la fermentation, la détermination du nombre ayant lieu directement dans le contenu du fermenteur.

2. Procédé suivant la revendication 1, dans lequel on détermine en plus un deuxième nombre de bactéries ne prenant pas le gram dans au moins une partie du contenu du fermenteur.

3. Procédé suivant la revendication 2, dans lequel on détermine, à partir du rapport du premier nombre et du deuxième nombre, s'il y a un déséquilibre de la fermentation.

4. Installation (10, 20) de production de biogaz, comprenant un fermenteur (11), conformée pour la détection d'un déséquilibre de la fermentation dans le fermenteur (11), comprenant un dispositif (12 ... 16, 21) de capteur, disposé dans le fermenteur (11), pour déterminer un premier nombre de bactéries prenant le gram dans au moins une partie du contenu du fermenteur au moyen de la spectroscopie infrarouge.

5. Installation (10, 20) de production de biogaz suivant la revendication 4, dans laquelle le dispositif (12 ... 16, 21) de capteur est conformé, pour déterminer, en outre, un deuxième nombre de bactéries ne prenant pas le gram dans au moins une partie du contenu du fermenteur.

6. Installation (10, 20) suivant la revendication 4 ou 5, dans laquelle le dispositif (12 ... 16, 21) de capteur comprend une sonde (21) de réflexion à l'intérieur du fermenteur (11).

7. Installation (10, 20) suivant l'une des revendications 4 à 6, dans laquelle le dispositif (12 ... 16, 21) de capteur est conformé pour effectuer la spectroscopie infrarouge dans le domaine du spectre de l'infrarouge proche.

8. Installation (10, 20) suivant l'une des revendications 4 à 7, dans laquelle il est prévu des moyens d'une exploitation statistique des données spectroscopiques.
